# EUROPEAN PATENT APPLICATION

(11) **EP 1 982 697 A1**
(43) Date of publication of application: **22.10.2008**
(21) Application number: 06768298.9
(22) Date of filing: 19.07.2006
(51) Int. Cl.: A61K 9/107, A61K 8/04, A61K 8/20, A61K 8/81, A61K 8/92, A61K 47/04, A61K 47/14, A61K 47/32

(54) **EMULSION LOTION**

(30) Priority: 03.10.2005 JP 2005290121
(71) Applicant: TAISHO PHARMACEUTICAL CO., LTD, Tokyo 170-8633 (JP)
(72) Inventor: NISHIOKU, Yoshinori, Tokyo 170-8633 (JP); SUZUKI, Yasuyuki, okxo 170-8633 (JP); MARUMOTO, Aya, Tokyo 170-8633 (JP); KONDO, Osamu, Tokyo 170-8633 (JP)
(74) Representative: HOFFMANN EITLE
(86) International application number: PCT/JP2006/314272
(87) International publication number: WO 2007/039974

(57) **Abstract**

An emulsion lotion composition including
a) a partially hydrolyzed polyvinyl alcohol having a degree of saponification of 70 to 96 mol %,
b) a carboxyvinyl polymer,
c) an oil component at not greater than 40 mass % relative to the total compositional amount, at least 60 mass % of the contained oil component being a liquid oil, and
d) an inorganic salt,

the pH being 3.5 to 8.5, and the viscosity at 25°C being 30 mPa·s to 310 mPa·s by oscillation viscometer, is an emulsion lotion composition that can be stored stably for a long period of time without adding a surfactant, has an excellent feeling when used, and can be prepared simply. This enables it to be used as an external preparation such as a pharmaceutical or a cosmetic.

## Description

### TECHNICAL FIELD

The present invention relates to an emulsion lotion that has little stimulation to the skin, is safe, can be stored stably for a long period of time, and gives an excellent feeling when used.

### BACKGROUND ART

An external preparation such as an ointment, a cream, or a lotion is generally used for the treatment of skin disease. Among lotions, an emulsion lotion has come into wide use since a lipid-soluble drug and a water-soluble drug can be added at the same time, the amount used may be a small amount compared with an ointment, the method of use is easy, the feeling upon application is excellent, it is convenient for using on areas where there is hair, and so on (ref. Nonpatent Document 1).

Furthermore, since an emulsion lotion composition is generally a liquid form, it spreads and conforms to the surface of the skin, and from the viewpoint of supply of moisture to the stratum corneum, since there are appropriate amounts of moisture and oil component, it is effective for rough skin or dry skin.

Conventionally, a stable emulsion lotion composition is obtained by the use of various viscous agents and surfactants such as a carboxyvinyl polymer, sodium carboxymethylcellulose, hydroxypropylcellulose, and xanthan gum.

However, it has been suggested that when used directly on the skin, as for a pharmaceutical or a cosmetic, a surfactant is one cause of stimulation to the skin (ref. Nonpatent Documents 2 and 3).

Because of this, for the purpose of further enhancing the safety an attempt has been made to obtain a stable emulsion lotion composition without using a surfactant.

For example, an emulsion composition (emulsion lotion) employing an alkyl-modified carboxyvinyl polymer such as an acrylic acid/alkyl methacrylate copolymer has been disclosed (ref. Patent Document 1). However, the application of an alkyl-modified carboxyvinyl polymer is restricted due to there being cases in which the use thereof is not allowed in a pharmaceutical, etc.

[Patent Document 1] Japanese Patent Application Laid-open No. 9-19631
[Nonpatent Document 1] The Japanese Pharmacopoeia Fourteenth Edition, Commentary, A117-A120 (2001)
[Nonpatent Document 2] Tamie Suzuki et al., Journal of the Japanese Society for Cutaneous Health, 51, 177-183 (2004) [Nonpatent Document 3] Hitoshi Tatsumi et al., Hifu (Skin), 33 (11), 31-38 (1991)

### DISCLOSURE OF THE INVENTION

### PROBLEMS TO BE SOLVED BY THE INVENTION

It is an object of the present invention.to provide an emulsion lotion composition that can be stored stably for a long period of time without adding a surfactant, has an excellent feeling when used, and can be prepared simply.

### MEANS FOR SOLVING THE PROBLEMS

As a result of an intensive investigation by the present inventors in order to solve the problems, it has been found that an emulsion lotion composition that has little stimulation to the skin, is safe, can be stored stably for a long period of time, and has an excellent feeling when used can be obtained simply by adding, to an O/W emulsion composition obtained by adding a specific oil component at not greater than 40 mass % relative to the total compositional amount to a partially hydrolyzed polyvinyl alcohol having a degree of saponification of 70 to 96 mol % and a carboxyvinyl polymer and adjusting the pH to 3.5 to 8.5, an inorganic salt so as to adjust the viscosity to a specific range, and the present invention has thus been accomplished.

That is, the present invention is:
(1) An emulsion lotion including:
   a) a partially hydrolyzed polyvinyl alcohol having a degree of saponification of 70 to 96 mol %,
   b) a carboxyvinyl polymer,
   c) an oil component at not greater than 40 mass % relative to the total compositional amount, equal to or greater than 60 mass % of the contained oil component being liquid oil, and
   d) an inorganic salt,
   the pH being 3.5 to 8.5, and the viscosity at 25°C being 30 mPa·s to 310 mPa·s by oscillation viscometer.
(2) The emulsion lotion according to (1), wherein the inorganic salt is one type or two or more types selected from magnesium chloride, sodium chloride, ammonium chloride, potassium chloride, and calcium chloride.
(3) The emulsion lotion according to (1), containing substantially no surfactant.
(4) The emulsion lotion according to (1), wherein the liquid oil is polar liquid oil.
(5) The emulsion lotion according to (4), wherein the polar liquid oil is synthetic oil having an IOB value of equal to or greater than 0.1.
(6) The emulsion lotion according to (4), wherein the polar liquid oil is vegetable oil having a fatty acid triglyceride as a main component.
(7) The emulsion lotion according to any one of (1) to (6), which is a composition for external use.
(8) The emulsion lotion according to any one of (1) to (6), further including a polyol.

### EFFECTS OF THE INVENTION

In accordance with the present invention, an emulsion lotion that can be stored stably for a long period of time, has little stimulation to the skin, and has high safety can be obtained. Furthermore, in accordance with the present invention, since the viscosity can be adjusted by addition of an inorganic salt, it becomes possible to change the feeling when used according to the intended application.

### BEST MODE FOR CARRYING OUT THE INVENTION

In the present invention, 'partially hydrolyzed polyvinyl alcohol' means a polyvinyl acetate obtained by polymerization of vinyl acetate being partially hydrolyzed; for example, there are several types having different viscosities, such as those with a viscosity of a 4% aqueous solution of 4.8 to 5.8 mPa·s, 20.5 to 24.5 mPa·s, 27.0 to 33.0 mPa·s, 40.0 to 46.0 mPa·s, etc., but in the present invention a stable emulsion lotion composition can be produced by the use of a generally used one having a degree of saponification of 70 to 96 mol %, irrespective of the molecular weight and viscosity thereof. From the viewpoint of preparations and the feeling when used, a partially hydrolyzed polyvinyl alcohol having a degree of saponification of 78 to 96 mol % is preferable, and a partially hydrolyzed polyvinyl alcohol having a degree of saponification of 85 to 90 mol % is particularly preferable. A polyvinyl alcohol having a degree of saponification of equal to or greater than 97 mol %, which is generally called a completely hydrolyzed polyvinyl alcohol, is differentiated from the partially hydrolyzed polyvinyl alcohol in the present invention.

The content of the partially hydrolyzed polyvinyl alcohol is 0.1 to 4.0 mass % relative to the total amount of the composition, and in terms of preparations it is preferably 0.2 to 3.0 mass %. The content of the partially hydrolyzed polyvinyl alcohol may be adjusted as appropriate according to other components and the intended application and, for example, when it is used as an external lotion for the skin, it is preferably added at 0.5 to 2.0 mass % of the total amount of the formulation.

In the present invention, 'carboxyvinyl polymer' means a copolymer of acrylic acid; for example, there are several types having different viscosities, such as those with a viscosity of a 0.5% aqueous solution of 4000 to 10000 mPa·s, 40000 to 60000 mPa·s, etc., and they may be selected for use as appropriate according to the intended application.

The content of the carboxyvinyl polymer is 0.1 to 3.0 mass % relative to the total amount of the composition, and it is preferably 0.3 to 2.0 mass % in terms of preparations. When it is in this range, an emulsion lotion having a good feeling when used and high stability can be obtained.

The content of an oil component in the present invention is not greater than 40 mass % relative to the total amount of the lotion, and equal to or greater than 60 mass % of the contained oil component is liquid oil. When the oil component is added at greater than 40 mass % relative to the total amount of the lotion, the oil component separates, and an emulsion cannot be prepared. Furthermore, when the proportion of the liquid oil in the oil component is less than 60 mass %, properties become poor and the stability is decreased, and it is therefore necessary for the proportion of the liquid oil in the oil component to be at least 60 mass %. Moreover, in terms of preparations, the amount of the oil component added is more preferably not greater than 35 mass % relative to the total amount of the lotion.

With regard to the 'oil component' referred to in the present invention, either liquid oil or a solid fat may be used, and liquid oil is preferable. The 'liquid oil' referred to here means either a nonpolar oil or a polar oil that is in a liquid state at 25°C, and is preferably a polar liquid oil. The polar liquid oil is preferably synthetic oil having an IOB value (Inorganic Organic Balance value) of equal to or greater than 0.1, or a vegetable oil having a fatty acid triglyceride as a main component.

The IOB value referred to here is a numerical value given by inorganic nature value/organic nature value, in which the organic nature value and the inorganic nature value are determined based on a method described in the literature [Yoshio Kouda, 'Organic Concepts - Basics and Applications', Sankyo Publishing (1984)].

With regard to the oil component, examples of the nonpolar liquid oil include liquid paraffin and synthetic squalane, examples of the polar liquid oil include a medium chain fatty acid triglyceride, octyldodecyl myristate, cetyl isooctanoate, isopropyl myristate, polyglyceryl-2 triisostearate, caprylic/capric acid triglyceride, triethylhexanoin, diisopropyl sebacate, tri-2-ethylhexyl citrate, diisopropyl adipate, propylene glycol monocaprylate, diethyl sebacate, hexyldecyl isostearate, isopropyl palmitate, isopropyl linoleate, ethyl linoleate, triethylhexanoin, propylene glycol dicaprylate, butylethyl propanediyl ethylhexanoate, isocetyl myristate, butyl myristate, cetyl ethylhexanoate, ethyl oleate, ethylhexyl palmitate, isopropyl isostearate, ethylhexyl stearate, octyldodecyl neopentanoate, propylene glycol dicaprate, pentaerythrityl tetraethylhexanoate, propylene glycol dicaprylate, trimethylolpropane triethylhexanoate, trimethylolpropane triisostearate, dibutyloctyl sebacate, diisostearyl malate, isocetyl stearate, isostearyl palmitate, octyldodecanol, hexyldecanol, oleyl alcohol, isostearyl alcohol, olive oil, almond oil, safflower oil, castor oil, corn oil, avocado oil, persic oil, kukui nut oil, grape seed oil, pistachio seed oil, hazelnut oil, macadamia nut oil, meadowfoam oil, rose hip oil, sunflower oil, soybean oil, jojoba oil, and oleic acid, and examples of the solid fat include solid paraffin, white vaseline, myristyl myristate, cetyl palmitate, stearyl alcohol, cetyl alcohol, cetostearyl alcohol, behenyl alcohol, stearic acid, myristic acid, hard fat, jojoba wax, and cacao butter.

The oil component is particularly preferably a liquid synthetic oil, having an IOB value of at least 0.1, selected from cetyl isooctanoate, isopropyl myristate, polyglyceryl-2 triisostearate, caprylic/capric acid triglyceride, triethylhexanoin, diisopropyl sebacate, tri-2-ethylhexyl citrate, diisopropyl adipate, propylene glycol monocaprylate, diethyl sebacate, hexyldecyl isostearate, isopropyl palmitate, isopropyl linoleate, ethyl linoleate, triethylhexanoin, propylene glycol dicaprylate, butylethyl propanediyl ethylhexanoate, isocetyl myristate, butyl myristate, cetyl ethylhexanoate, ethyl oleate, ethylhexyl palmitate, isopropyl isostearate, ethylhexyl stearate, octyldodecyl neopentanoate, propylene glycol dicaprate, pentaerythrityl tetraethylhexanoate, propylene glycol dicaprylate, trimethylolpropane triethylhexanoate, trimethylolpropane triisostearate, dibutyloctyl sebacate, and diisostearyl malate, or a liquid vegetable oil having a fatty acid triglyceride as a main component, selected from olive oil, almond oil, safflower oil, castor oil, corn oil, avocado oil, persic oil, kukui nut oil, grape seed oil, pistachio seed oil, hazelnut oil, macadamia nut oil, meadowfoam oil, rose hip oil, sunflower oil, and soybean oil.

One type of these oil components may be used, or two or more types thereof may be used in combination.

The pH of the emulsion lotion of the present invention is preferably adjusted to 3.5 to 8.5. When the pH value is less than 3.5, emulsification stability becomes poor, and a target emulsion lotion cannot be obtained. Furthermore, when the emulsion lotion of the present invention is used on the skin, if the pH exceeds 8.5, there is a possibility of stimulation of the skin occurring, and it is not suitable for use on the skin. From the viewpoint of preparations, it is more preferable to adjust the pH to 4.5 to 8.0.

A pH adjusting agent is not particularly limited, and a basic compound that is normally added to a pharmaceutical or a cosmetic may be used. Examples thereof include diisopropanolamine, triisopropanolamine, sodium hydroxide, monoethanolamine, diethanolamine, triethanolamine, triethylamine, potassium hydroxide, and sodium citrate. When adjusting the pH, one type of these pH adjusting agents may be used or two or more types thereof may be used in combination.

With regard to the emulsion lotion of the present invention, the viscosity at about 25°C by oscillation viscometer is adjusted so as to be in the range of 30 mPa·s to 310 mPa·s by adding an inorganic salt to an O/W emulsion composition as an intermediate composition. When the viscosity value is less than 30 mPa·s, emulsification stability becomes poor, and a target emulsion lotion cannot be obtained. On the other hand, when the viscosity value exceeds 310 mPa·s, the composition does not attain a lotion state and is therefore difficult to use. Furthermore, in order to obtain stability for a longer period of time, it is preferable to adjust the viscosity so as to be in the range of 50 mPa·s to 310 mPa·s.

The inorganic salt referred to in the present invention is an equimolar mixture containing an inorganic acid and an inorganic base, and an inorganic salt normally added to a pharmaceutical or a cosmetic may be used. Examples of the inorganic salt include sodium chloride, potassium chloride, ammonium chloride, calcium chloride, and magnesium chloride, and it is preferable to use one type or two or more types selected from sodium chloride, potassium chloride, and magnesium chloride.

In the present invention, two or more types of the inorganic salt may be used in combination.

By further adding a polyol to the emulsion lotion of the present invention, the present lotion is given an excellent feeling when used. The polyol that can be used in the present invention is not particularly limited; examples thereof include 1,3-butylene glycol, propylene glycol, dipropylene glycol, glycerin, and polyethylene glycol, and one type or two or more types in combination of these polyols may be used.

Furthermore, in the present invention, 'containing substantially no surfactant' means that a surfactant is not added or a surfactant is contained in a small amount that does not allow an interfacial activation effect to be exhibited sufficiently.

The surfactant referred to here means a compound that has a hydrophilic group and a hydrophobic group, that normally dissolves in water, greatly changes properties such as surface tension, and at the same time forms an assembly such as a micelle in water.

The emulsion lotion of the present invention may be produced in accordance with a standard emulsion production process. For example, the carboxyvinyl polymer is dispersed in purified water and heated, the partially hydrolyzed polyvinyl alcohol is separately dissolved in heated purified water, and the two aqueous solutions are combined to give an aqueous phase. On the other hand, the oil component (if necessary, a polyol may be added as appropriate) is heated, thus giving an oil phase. The oil phase is added to the aqueous phase, purified water is further added thereto and stirring is carried out until uniform, and subsequently the mixture is cooled to room temperature while stirring, thus giving an O/W emulsion composition as an intermediate composition. The pH adjusting agent is added by dissolving an appropriate amount thereof in the oil phase or the final purified water. An appropriate amount of the inorganic salt is dissolved in purified water and added to this intermediate O/W emulsion composition, thus giving a target emulsion lotion.

The emulsion lotion thus simply prepared can be stored stably for a long period of time, and since it is not necessary to add a surfactant, it can be a lotion that has little stimulation when used on the skin. Furthermore, it spreads easily, and an excellent feeling when used is obtained.

Moreover, it is possible to add to the emulsion lotion of the present invention in a range that does not impair a desired effect an active ingredient and various types of base components that can be contained in a pharmaceutical, a quasi drug, and a cosmetic.

Examples of the active ingredient include an antiinflammatory agent (indomethacin, piroxicam, diclofenac sodium, felbinac, hydrocortisone acetate, dexamethasone acetate, and so on), an analgesic, an antihistamine (diphenhydramine, chlorpheniramine maleate, isothipendyl hydrochloride, and so on), a local anesthetic (lidocaine, procaine, dibucaine, lidocaine hydrochloride, procaine hydrochloride, dibucaine hydrochloride, and so on), a tissue repair agent, an antipruritic (crotamiton, and so on), urea, a moisturizing agent (hyaluronic acid, ceramide, and so on), an algefacient (menthol, camphor, and so on), a vitamin and a derivative thereof (vitamin A, vitamin B, vitamin C, vitamin E, and so on), an angiotonic (tetrahydrozoline hydrochloride, phenylephrine hydrochloride, and so on), a skin-lightening agent, an antibacterial agent, an antiallergic agent, an antiviral agent, an antifungal agent (miconazole nitrate, terbinafine hydrochloride, bifonazole, neticonazole hydrochloride, butenafine hydrochloride, liranaftate, luliconazole, and so on), an oxygen remover, a UV absorber, and a UV scattering agent.

Furthermore, examples of the base component include a solubilizing agent such as a lower alcohol (ethanol, isopropanol, and so on), a hydrocarbon, a wax component, an antioxidant (dibutylhydroxytoluene, and so on), an emulsion stabilizer, a gelator (methylcellulose, hydroxyethylcellulose, hydroxypropylcellulose, hydroxypropylmethylcellulose, sodium alginate, xanthan gum, polyvinylpyrrolidone, and so on), a thickener, an adhesive, extracts from various types of animals and plants, an antiseptic (parahydroxybenzoate esters, benzyl alcohol, phenoxyethanol, and so on), a chelating agent (sodium edetate, and so on), a fragrance, a colorant, and a liquefied gas.

### Examples

The present invention is explained in further detail below by reference to examples, comparative examples, and test examples. Unless otherwise specified, Carbopol 980 manufactured by Noveon was used as the carboxyvinyl polymer, PANACET 810 manufactured by NOF Corporation was used as the medium chain fatty acid triglyceride, the magnesium chloride was a product conforming to Japanese Standards for Pharmaceutical Ingredients, and Gohsenol EG-05 (product name; degree of saponification 86.5 to 89.0 mol %) manufactured by The Nippon Synthetic Chemical Industry Co., Ltd. was used as the partially hydrolyzed polyvinyl alcohol. Furthermore, the viscosity was measured using a Type VM-100 oscillation viscometer and a PR-100-L probe (manufactured by Yamaichi Electronics Co., Ltd.) at about 25°C.

### [Example 1]

| | |
|---|---|
| Medium chain fatty acid triglyceride | 13 mass % |
| Carboxyvinyl polymer | 0.5 mass % |
| Partially hydrolyzed polyvinyl alcohol | 1 mass % |
| 1,3-Butylene glycol | 10 mass % |
| Diisopropanolamine | appropriate amount |
| Magnesium chloride | 0.12 mass % |
| Purified water | total 100 mass % |
| pH 4.80 | |

0.5 g of carboxyvinyl polymer was dispersed in 25 g of purified water and heated to about 70°C. Separately, 1.0 g of partially hydrolyzed polyvinyl alcohol was dissolved in 25 g of purified water heated to about 70°C, and the two aqueous solutions were combined, thus giving an aqueous phase. Separately, 13 g of medium chain fatty acid triglyceride and 10 g of 1,3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. 0.12 g of magnesium chloride was dissolved in the remaining purified water and added to the O/W emulsion composition, thus giving a lotion having a viscosity by oscillation viscometer of 177 mPa·s.

### [Example 2]

| | |
|---|---|
| Hexyldecanol | 4 mass % |
| Isopropyl myristate | 12 mass % |
| Carboxyvinyl polymer | 0.8 mass % |
| Partially hydrolyzed polyvinyl alcohol | 1.2 mass % |
| Glycerin | 5 mass % |
| Sodium hydroxide | appropriate amount |
| Sodium chloride | 0.09 mass % |
| Purified water | total 100 mass % |
| pH 6.20 | |

0.8 g of carboxyvinyl polymer was dispersed in 35 g of purified water and heated to about 70°C. Separately, 1.2 g of partially hydrolyzed polyvinyl alcohol was dissolved in 15 g of purified water heated to about 70°C, and the two aqueous solutions were combined, thus giving an aqueous phase. Separately, 12 g of isopropyl myristate, 4 g of hexyldecanol, and 5 g of glycerin were heated to about 70°C, thus giving an oil phase. The oil phase was added to the aqueous phase and stirred, subsequently a solution of sodium hydroxide (appropriate amount) in 10 g of purified water was added thereto, and the mixture was cooled to room temperature while further stirring until uniform, thus giving an O/W emulsion composition. 0.09 g of sodium chloride was dissolved in the remaining purified water and added to the O/W emulsion composition, thus giving a lotion having a viscosity by oscillation viscometer of 310 mPa·s. [Example 3]

| | |
|---|---|
| Isopropyl myristate | 10 mass % |
| Stearyl alcohol | 2 mass % |
| Carboxyvinyl polymer | 0.5 mass % |
| Partially hydrolyzed polyvinyl alcohol | 0.8 mass % |
| Triethanolamine | appropriate amount |
| Sodium chloride | 0.26 mass % |
| Purified water | total 100 mass % |
| pH 4.60 | |

0.5 g of carboxyvinyl polymer was dispersed in 20 g of purified water and heated to about 70°C. Separately, 0.8 g of partially hydrolyzed polyvinyl alcohol was dissolved in 20 g of purified water heated to about 70°C, and the two aqueous solutions were combined, thus giving an aqueous phase. Separately, 10 g of isopropyl myristate and 2 g of stearyl alcohol were heated to about 70°C, thus giving an oil phase. The oil phase was added to the aqueous phase and stirred, subsequently a solution of triethanolamine (appropriate amount) in 10 g of purified water was added thereto, and the mixture was cooled to room temperature while further stirring until uniform, thus giving an O/W emulsion. 0.26 g of sodium chloride was dissolved in the remaining purified water and added to the O/W emulsion, thus giving a lotion having a viscosity by oscillation viscometer of 80 mPa·s.

### [Example 4]

| | |
|---|---|
| Medium chain fatty acid triglyceride | 12 mass % |
| Squalane | 2 mass % |
| Carboxyvinyl polymer | 0.5 mass % |
| Partially hydrolyzed polyvinyl alcohol | 1 mass % |
| Polyethylene glycol 400 | 12 mass % |
| Diisopropanolamine | appropriate amount |
| Magnesium chloride | 0.08 mass %. |
| Ammonium chloride | 0.09 mass % |
| Purified water | total 100 mass % |
| pH 4.65 | |

0.5 g of carboxyvinyl polymer was dispersed in 25 g of purified water and heated to about 70°C. Separately, 1.0 g of partially hydrolyzed polyvinyl alcohol were dissolved in 25 g of purified water heated to about 70°C, and the two aqueous solutions were combined, thus giving an aqueous phase. Separately, 12 g of medium chain fatty acid triglyceride, 2 g of squalane, and 12 g of polyethylene glycol 400 were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. 0.08 g of magnesium chloride and 0.09 g of ammonium chloride were dissolved in the remaining purified water and added to the O/W emulsion composition, thus giving a lotion having a viscosity by oscillation viscometer of 57 mPa·s.

### [Example 5]

| | |
|---|---|
| Medium chain fatty acid triglyceride | 15 mass % |
| Carboxyvinyl polymer | 0.4 mass % |
| Hydroxypropylmethylcellulose | 0.05 mass % |
| Partially hydrolyzed polyvinyl alcohol | 1.2 mass % |
| 1,3-Butylene glycol | 10 mass % |
| Diisopropanolamine | appropriate amount |
| Ammonium chloride | 0.22 mass % |
| Purified water | total 100 mass % |
| pH 4.50 | |

0.4 g of carboxyvinyl polymer was dispersed in 25 g of purified water and heated to about 70°C. Separately, 1.2 g of partially hydrolyzed polyvinyl alcohol was dissolved in 15 g of purified water heated to 70°C. Furthermore, 0.05 g of hydroxypropylmethylcellulose was swollen in 10 g of purified water heated to 70°C, and the three aqueous solutions were combined, thus giving an aqueous phase. Separately, 15 g of medium chain fatty acid triglyceride and 10 g of 1,3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. 0.22 g of ammonium chloride was dissolved in the remaining purified water and added to the O/W emulsion composition thus giving a lotion having a viscosity by oscillation viscometer of 30 mPa·s.

### [Example 6]

| | |
|---|---|
| Isopropylmethylphenol | 0.1 mass % |
| dl-Camphor | 0.5 mass % |
| Cetyl alcohol | 1 mass % |
| Medium chain fatty acid triglyceride | 13 mass % |
| Carboxyvinyl polymer | 0.7 mass % |
| Partially hydrolyzed polyvinyl alcohol | 1 mass % |
| Polyethylene glycol 400 | 5 mass % |
| Sodium edetate | 0.1 mass % |
| Diisopropanolamine | appropriate amount |
| Magnesium chloride | 0.12 mass % |
| Purified water | total amount 100 mass % |
| pH 5.10 | |

0.7 g of carboxyvinyl polymer was dispersed in 30 g of purified water and heated to about 70°C. Separately, 1.0 g of partially hydrolyzed polyvinyl alcohol was dissolved in 15 g of purified water heated to about 70°C, and the two aqueous solutions were combined, thus giving an aqueous phase. Separately, 13 g of medium chain fatty acid triglyceride and 1 g of cetyl alcohol were heated to about 70°C, and 0.5 g of dl-camphor was dissolved therein, thus giving an oil phase. Furthermore, 5 g of polyethylene glycol 400 was heated to about 70°C, and 0.1 g of isopropylmethylphenol and diisopropanolamine (appropriate amount) were dissolved therein, thus giving a polyol phase. The oil phase was added to the aqueous phase, the polyol phase was further added thereto, subsequently a solution of 0.1 g of sodium edetate in 10 g of purified water was added thereto, and this was stirred until uniform and cooled to room temperature while stirring, thus giving an O/W emulsion composition. 0.12 g of magnesium chloride was dissolved in the remaining purified water and added to the O/W emulsion composition, thus giving a lotion having a viscosity by oscillation viscometer of 105 mPa·s.

### [Example 7]

| | |
|---|---|
| Dexamethasone acetate | 0.025 mass % |
| Retinol palmitate | 200000 I.U. |
| 1-Menthol | 0.25 mass % |
| Medium chain fatty acid triglyceride | 12 mass % |
| Liquid paraffin | 1 mass % |
| Carboxyvinyl polymer | 0.6 mass % |
| Partially hydrolyzed polyvinyl alcohol | 1.2 mass % |
| 1,3-Butylene glycol | 8 mass % |
| Ethyl parahydroxybenzoate | 0.1 mass % |
| Propyl parahydroxybenzoate | 0.1 mass % |
| Dibutylhydroxytoluene | 0.1 mass % |
| Sodium hydroxide | appropriate amount |
| Magnesium chloride | 0.09 mass % |
| Purified water | total 100 mass % |
| pH 5.45 | |

0.6 g of carboxyvinyl polymer was dispersed in 25 g of purified water and heated to about 70°C. Separately, 1.2 g of partially hydrolyzed polyvinyl alcohol was dissolved in 25 g of purified water heated to about 70°C, and the two aqueous solutions were combined, thus giving an aqueous phase. Separately, 12 g of medium chain fatty acid triglyceride and 1 g of liquid paraffin were heated to about 70°C, and 200000 I.U. of retinol palmitate, 0.25 g of 1-menthol, 0.1 g of dibutylhydroxytoluene, 0.1 g of ethyl parahydroxybenzoate, and 0.1 g of propyl parahydroxybenzoate were dissolved therein, thus giving an oil phase. Furthermore, 8 g of 1,3-butylene glycol was heated to about 70°C, and 0.025 g of dexamethasone acetate was dissolved therein, thus giving a polyol phase. The oil phase was added to the aqueous phase, the polyol phase was further added thereto, and subsequently a solution of sodium hydroxide (appropriate amount) in 5 g of purified water was added thereto. After stirring until uniform, the mixture was cooled to room temperature while stirring, thus giving an O/W emulsion composition. 0.09 g of magnesium chloride was dissolved in the remaining purified water and added to the O/W emulsion composition, thus giving a lotion having a viscosity by oscillation viscometer of 162 mPa·s.

### [Example 8]

| | |
|---|---|
| Miconazole nitrate | 1.0 mass % |
| Medium chain fatty acid triglyceride | 14 mass % |
| Carboxyvinyl polymer | 0.6 mass % |
| Partially hydrolyzed polyvinyl alcohol | 1.2 mass % |
| 1,3-Butylene glycol | 10 mass % |
| Diisopropanolamine | appropriate amount |
| Ammonium chloride | 0.15 mass % |
| Purified water | total 100 mass % |
| pH 4.65 | |

0.6 g of carboxyvinyl polymer is dispersed in 30 g of purified water and heated to about 70°C. Furthermore, 1.2 g of partially hydrolyzed polyvinyl alcohol is dissolved in 15 g of purified water heated to about 70°C. The two aqueous solutions were combined, thus giving an aqueous phase. Separately, 14 g of medium chain fatty acid triglyceride and 10 g of 1,3-butylene glycol were heated to about 70°C, and diisopropanolamine (appropriate amount) and 1.0 g of miconazole nitrate were dissolved therein, thus giving an oil phase. The oil phase was added to the aqueous phase, and this was stirred until uniform and subsequently cooled to room temperature while stirring, thus giving an O/W emulsion composition. 0.15 g of ammonium chloride was dissolved in the remaining purified water and added to the O/W emulsion composition, thus giving a lotion having a viscosity by oscillation viscometer of 73 mPa·s.

### [Example 9]

| | |
|---|---|
| Indomethacin | 1.0 mass % |
| 1-Menthol | 1.5 mass % |
| Medium chain fatty acid triglyceride | 12 mass % |
| Carboxyvinyl polymer | 0.7 mass % |
| Partially hydrolyzed polyvinyl alcohol | 1.0 mass % |
| 1,3-Butylene glycol | 10 mass % |
| Diisopropanolamine | appropriate amount |
| Ammonium chloride | 0.16 mass % |
| Purified water | total 100 mass % |
| pH 4.70 | |

0.7 g of carboxyvinyl polymer was dispersed in 30 g of purified water and heated to about 70°C. Separately, 1.0 g of partially hydrolyzed polyvinyl alcohol was dissolved in 15 g of purified water heated to about 70°C, and the two aqueous solutions were combined, thus giving an aqueous phase. Separately, 12 g of medium chain fatty acid triglyceride and 10 g of 1,3-butylene glycol were heated to about 70°C, and 1.0 g of indomethacin, 1.5 g of 1-menthol, and diisopropanolamine (appropriate amount) were dissolved therein, thus giving an oil phase. The oil phase was added to the aqueous phase, and this was stirred until uniform and subsequently cooled to room temperature while stirring, thus giving an O/W emulsion composition. 0.16 g of ammonium chloride was dissolved in the remaining purified water and added to the O/W emulsion composition, thus giving a lotion having a viscosity by oscillation viscometer of 90 mPa·s.

### [Example 10]

| | |
|---|---|
| Urea | 20 mass % |
| Lidocaine | 0.3 mass % |
| Isopropylmethylphenol | 0.1 mass % |
| dl-Camphor | 0.5 mass % |
| Medium chain fatty acid triglyceride | 12 mass % |
| Liquid paraffin | 1 mass % |
| Carboxyvinyl polymer | 0.6 mass % |
| Partially hydrolyzed polyvinyl alcohol | 1.2 mass % |
| 1,3-Butylene glycol | 8 mass % |
| Ethyl parahydroxybenzoate | 0.1 mass % |
| Propyl parahydroxybenzoate | 0.1 mass % |
| Dibutylhydroxytoluene | 0.1 mass % |
| Diisopropanolamine | appropriate amount |
| Sodium chloride | 0.26 mass % |
| Purified water | total 100 mass % |
| pH 4.65 | |

0.6 g of carboxyvinyl polymer was dispersed in 30 g.of purified water and dissolved by heating to about 70°C. Separately, 1.2 g of partially hydrolyzed polyvinyl alcohol was dissolved in 20 g of purified water heated to about 70°C, and 20 g of urea was further dissolved therein. The two aqueous solutions were combined, thus giving an aqueous phase. Separately, 12 g of medium chain fatty acid triglyceride and 1 g of liquid paraffin were heated to about 70°C, and 0.3 g of lidocaine, 0.5 g of dl-camphor, 0.1 g of dibutylhydroxytoluene, and 0.1 g of propyl parahydroxybenzoate were dissolved therein, thus giving an oil phase. Furthermore, 8 g of 1,3-butylene glycol was heated to about 70°C, and 0.1 g of isopropylmethylphenol was dissolved therein, thus giving a polyol phase. The oil phase was added to the aqueous phase, the polyol phase was further added thereto, and subsequently a solution of diisopropanolamine (appropriate amount) and 0.1 g of ethyl parahydroxybenzoate in remaining purified water was added thereto, and this was stirred until uniform and subsequently cooled to room temperature while stirring, thus giving an O/W emulsion composition. 0.26 g of sodium chloride was dissolved in the remaining purified water and added to the O/W emulsion composition, thus giving a lotion having a viscosity by oscillation viscometer of 62 mPa·s.

### [Example 11]

| | |
|---|---|
| Terbinafine hydrochloride | 1.0 mass % |
| Medium chain fatty acid triglyceride | 14 mass % |
| Carboxyvinyl polymer | 0.6 mass % |
| Partially hydrolyzed polyvinyl alcohol | 1.2 mass % |
| 1,3-Butylene glycol | 10 mass % |
| Diisopropanolamine | appropriate amount |
| Sodium chloride | 0.14 mass % |
| Purified water | total 100 mass % |
| pH 4.50 | |

0.6 g of carboxyvinyl polymer was dispersed in 30 g of purified water 30 g and heated to about 70°C. Separately, 1.2 g of partially hydrolyzed polyvinyl alcohol was dissolved in 15 g of purified water heated to about 70°C, and the two aqueous solutions were combined, thus giving an aqueous phase. Separately, 14 g of medium chain fatty acid triglyceride and 10 g of 1,3-butylene glycol were heated to about 70°C, and diisopropanolamine (appropriate amount) and 1.0 g of terbinafine hydrochloride were dissolved therein, thus giving an oil phase. The oil phase was added to the aqueous phase, and this was stirred until uniform and subsequently cooled to room temperature while stirring, thus giving an O/W emulsion composition. 0.14 g of sodium chloride was dissolved in the remaining purified water and added to the O/W emulsion composition, thus giving a lotion having a viscosity by oscillation viscometer of 100 mPa·s.

### [Example 12]

| | |
|---|---|
| Piroxicam | 0.5 mass % |
| 1-Menthol | 1.0 mass % |
| Medium chain fatty acid triglyceride | 12 mass % |
| Carboxyvinyl polymer | 0.6 mass % |
| Partially hydrolyzed polyvinyl alcohol | 1.0 mass % |
| 1,3-Butylene glycol | 12 mass % |
| Diisopropanolamine | appropriate amount |
| Ammonium chloride | 0.13 mass % |
| Purified water | total 100 mass % |
| pH 4.75 | |

0.6 g of carboxyvinyl polymer was dispersed in 30 g of purified water and heated to about 70°C. Separately, 1.0 g of partially hydrolyzed polyvinyl alcohol was dissolved in 15 g of purified water heated to about 70°C. The two aqueous solutions were combined, thus giving an aqueous phase. Separately, 12 g of medium chain fatty acid triglyceride and 12 g of 1,3-butylene glycol were heated to about 70°C, and diisopropanolamine (appropriate amount), 0.5 g of piroxicam, and 1.0 g of 1-menthol were dissolved therein, thus giving an oil phase. The oil phase was added to the aqueous phase, and this was stirred until uniform and subsequently cooled to room temperature while stirring, thus giving an O/W emulsion composition. 0.13 g of ammonium chloride was dissolved in the remaining purified water and added to the O/W emulsion composition, thus giving a lotion having a viscosity by oscillation viscometer of 94 mPa·s.

### Test Example 1 Prototype Test 1

0.5 g of carboxyvinyl polymer was dispersed in 25 g of purified water and heated to about 70°C. Separately, 1.0 g of partially hydrolyzed polyvinyl alcohol was dissolved in 25 g of purified water heated to about 70°C, and the two aqueous solutions were combined, thus giving an aqueous phase. Separately, 13 g of medium chain fatty acid triglyceride and 10 g of 1,3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. The pH was adjusted to 5 to 7. A solution of magnesium chloride (appropriate amount) in the remaining purified water was added to the O/W emulsion composition, thus giving a lotion of the present invention.

It was ascertained whether it was possible to finally prepare a uniform lotion having a good feeling when used by the same preparation method as for the lotion above Example 1 and Comparative Preparations 1 to 15 described in Table below. The ascertainment was carried out by three people. The results are given in Table 1. '○' in the table denotes that a uniform lotion having a good appearance and a good feeling when used was prepared. '×' denotes that a uniform preparation could not be obtained.

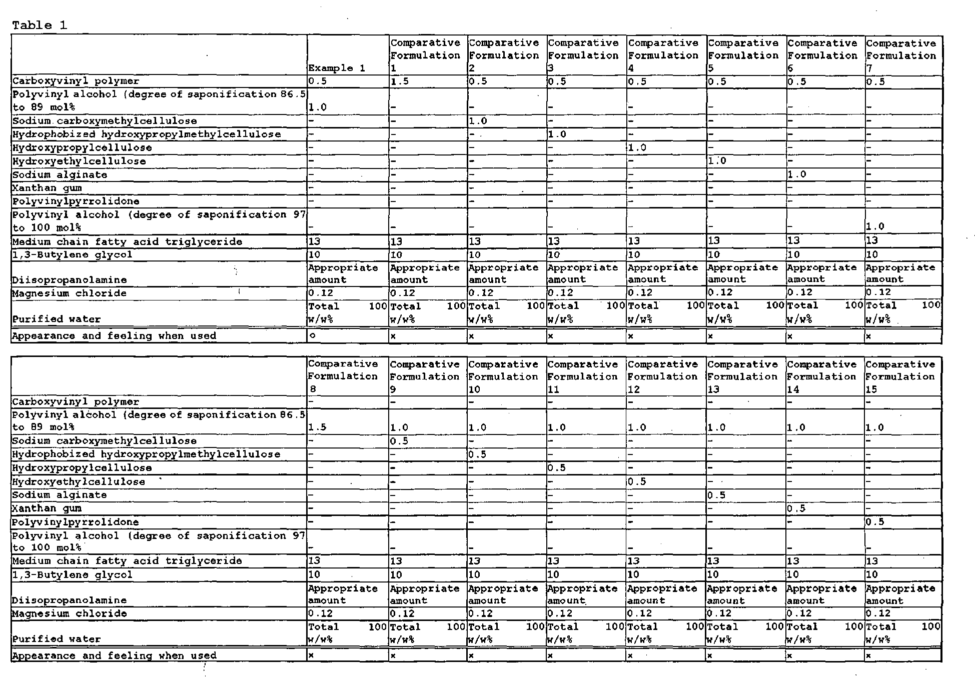

### Test Example 2 Change in viscosity according to amount of salt added

0.5 g of carboxyvinyl polymer was dispersed in 25 g of purified water and heated to about 70°C. Separately, 1.0 g of partially hydrolyzed polyvinyl alcohol was dissolved in 25 g of purified water heated to about 70°C, and the two aqueous solutions were combined, thus giving an aqueous phase. Separately, 13 g of medium chain fatty acid triglyceride and 10 g of 1,3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. The pH was adjusted to 5 to 7. This O/W emulsion composition was used as a standard, and solutions of 0.03 to 0.26 g of magnesium chloride in the remaining purified water were added thereto, thus preparing O/W emulsion composition samples.

The immediate appearance of the O/W emulsion compositions thus prepared was examined and the viscosity thereof was measured by oscillation viscometer. Furthermore, samples that were judged to be lotions were stored at 65°C for 3 days and 2 weeks (corresponding to storage at normal temperature for 3 years), the appearance after storage was examined, and the stability was assessed.

The results are given in the table. Samples having a viscosity exceeding 310 mPa·s had a cream-like appearance immediately after being produced. Samples that were prepared so as to have a viscosity of 20 mPa·s to 310 mPa·s could be judged to have the appearance of a lotion immediately after being produced, but the stability of the sample having a viscosity of 20 mPa·s could not be maintained. It was found that samples prepared so as to have a viscosity of 50 mPa·s to 310 mPa·s could maintain extremely good stability.

**Table 2**

| Content (%) | Viscosity (mPa·s) | Immediate appearance | Appearance after storage for 3 days | Appearance after storage for 2 weeks |
|---|---|---|---|---|
| 0.03 | 380 | White cream | - | - |
| 0.04 | 350 | White cream | - | - |
| 0.05 | 310 | White lotion | White lotion | White lotion |
| 0.06 | 260 | White lotion | White lotion | White lotion |
| 0.07 | 210 | White lotion | White lotion | White lotion |
| 0.10 | 140 | White lotion | White lotion | White lotion |
| 0.12 | 100 | White lotion | White lotion | White lotion |
| 0.14 | 70 | White lotion | White lotion | White lotion |
| 0.16 | 50 | White lotion | White lotion | White lotion |
| 0.18 | 40 | White lotion | White lotion | Slightly separated |
| 0.20 | 30 | White lotion | White lotion | Slightly separated |
| 0.26 | 20 | White lotion | Separated | Separated |

### Test Example 3 (Prototype Test 2: prototype test involving changing oil component)

In this test, O/W emulsion compositions for which the oil component was changed were prepared, and it was ascertained whether it was possible to prepare uniform preparations having a good feeling when used with these changed formulations. Subsequently, lotions were prepared using some of these oils, and it was ascertained whether it was possible to prepare a uniform preparation having a good feeling when used.

### Examination of O/W emulsion composition

0.5 g of carboxyvinyl polymer was dispersed in 25 g of purified water and heated to about 70°C, thus giving an aqueous solution. Separately, 1 g of partially hydrolyzed polyvinyl alcohol (degree of saponification 86.5 to 89.0 mol %) was dissolved in 25 g of purified water heated to about 70°C, thus giving an aqueous solution. The two aqueous solutions were combined, thus giving an aqueous phase. Separately, a mixture of 13 g of medium chain fatty acid triglyceride and 10 g of 1,3-butylene glycol was heated to about 70°C, and diisopropanolamine (appropriate amount) was dissolved therein, thus giving an oil phase. The oil phase was added to the aqueous phase, the remaining purified water was further added thereto, and this was stirred until uniform and subsequently cooled to room temperature while stirring, thus giving an O/W emulsion composition. The pH was adjusted to 5 to 7. This was used as a standard O/W emulsion composition.

### (1) Formulation with changed medium chain fatty acid triglyceride

Formulations obtained by changing the medium chain fatty acid triglyceride in the above standard formulation for each of paraffin, white vaseline (semisolid), liquid paraffin, synthetic squalane, cetyl palmitate, myristyl myristate, behenyl alcohol, cetyl alcohol, batyl alcohol, octyldodecyl myristate, cetyl isooctanoate, isopropyl myristate, polyglyceryl-2 triisostearate, caprylic/capric acid triglyceride, triethylhexanoin, diisopropyl sebacate, tri-2-ethylhexyl citrate, diisopropyl adipate, propylene glycol monocaprylate, olive oil, almond oil, and castor oil were made into creams by the same procedure as for the above standard formulation.

### (2) Formulation with component missing

A modified formulation in which the medium chain fatty acid triglyceride was removed from the above standard formulation was prepared in the same manner as for the above standard formulation.

In these changed formulations, it was ascertained whether it was possible to prepare a uniform formulation having a good feeling when used. Visual assessment was carried out by three people. The results are given in the table. The '65°C/2W' heading denotes properties examined after 2 weeks at 65°C.

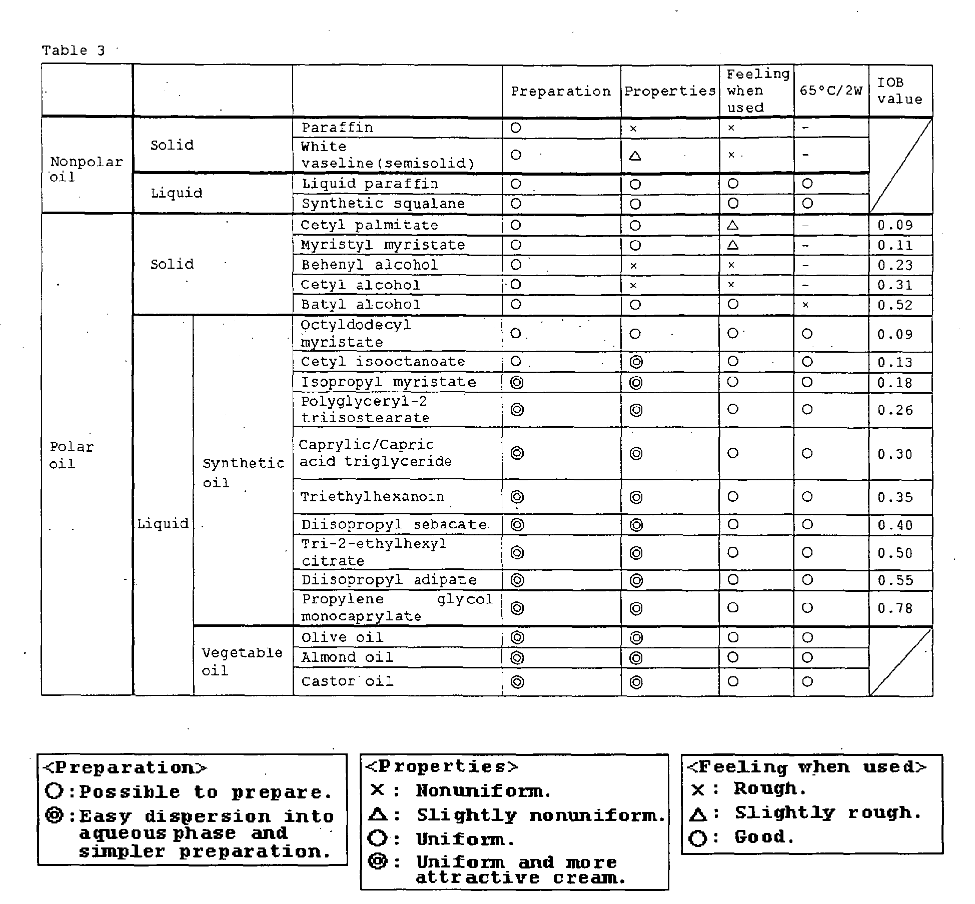

An emulsion composition could not be prepared from the formulation with the component missing.

### Assessment of lotion

0.5 g of carboxyvinyl polymer was dispersed in 25 g of purified water and heated to about 70°C. Separately, 1.0 g of partially hydrolyzed polyvinyl alcohol was dissolved in 25 g of purified water heated to about 70°C, and the two aqueous solutions were combined, thus giving an aqueous phase. Separately, 13 g of medium chain fatty acid triglyceride and 10 g of 1,3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. The pH was adjusted to 5 to 7. A solution of magnesium chloride (appropriate amount) in the remaining purified water was added to the O/W emulsion composition, thus giving a lotion of the present invention.

Formulations obtained by changing the medium chain fatty acid triglyceride in the above standard lotion formulation for each of white vaseline (semisolid), liquid paraffin, cetyl palmitate, myristyl myristate, batyl alcohol, octyldodecyl myristate, cetyl isooctanoate, isopropyl myristate, caprylic/capric acid triglyceride, triethylhexanoin, diisopropyl adipate, olive oil, almond oil, and castor oil were made into lotions by the same procedure as for the standard lotion formulation. The results are given in the table.

In these changed formulations, the properties after 2 weeks at 65°C were assessed. Visual assessment was carried out by three people. The results are given in Table 2. The '65°C/2W' heading denotes the properties after 2 weeks at 65°C. 'o' denotes that there was no change from those immediately after being prepared, 'Δ' denotes that there was no separation but crystallization of the oil component was observed and the feeling when used was degraded, and '×' denotes that separation was observed.

**Table 4**

| | | | Viscosity immediate after (mPa·s) | pH immediate after | 65°C-2W appearance |
|---|---|---|---|---|---|
| | | White vaseline (semisolid) | 228 | 4.95 | × |
| Liquid | | Liquid paraffin | 286 | 5.05 | ○ |
| Solid | | Myristyl myristate | 274 | 4.90 | Δ |
| | | Batyl alcohol | 290 | 4.75 | Δ |
| Liquid | Synthetic oil | Octyldodecyl myristate | 233 | 4.98 | ○ |
| | | Cetyl isooctanoate | 251 | 5.07 | ○ |
| | | Isopropyl myristate | 260 | 5.05 | ○ |
| | | Caprylic/Capric acid triglyceride | 247 | 5.04 | ○ |
| | | Triethylhexanoin | 279 | 5.07 | ○ |
| | | Diisopropyl adipate | 235 | 5.03 | ○ |
| | Vegetable oil | Olive oil | 297 | 5.09 | ○ |
| | | Almond oil | 280 | 5.08 | ○ |
| | | Castor oil | 289 | 5.21 | ○ |

### Test Example 4 (Prototype Test 3: test involving changing content)

In this test, O/W emulsion compositions in which the content of each component was changed were prepared, and for these changed formulations it was ascertained whether it was possible to prepare a uniform preparation having a good feeling when used. Following this, lotions in which the content of each component was changed were prepared, and it was ascertained whether it was possible to prepare a uniform formulation having a good feeling when used.

### Method for preparing O/W emulsion composition

0.5 g of carboxyvinyl polymer was dispersed in 25 g of purified water and heated to about 70°C, thus giving an aqueous solution. Furthermore, 1 g of partially hydrolyzed polyvinyl alcohol (degree of saponification 86.5 to 89.0 mol %) was dissolved in 25 g of purified water heated to about 70°C, thus giving an aqueous solution. The two aqueous solutions were combined, thus giving an aqueous phase. Separately, a mixture of 13 g of medium chain fatty acid triglyceride and 10 g of 1,3-butylene glycol was heated to about 70°C, and diisopropanolamine (appropriate amount) was dissolved therein, thus giving an oil phase. The oil phase was added to the aqueous phase, the remaining purified water was further added thereto, and this was stirred until uniform and subsequently cooled to room temperature while stirring, thus giving an O/W emulsion composition. This was used as a standard formulation. The pH was adjusted to 5 to 7.

### (1) Test involving changing content of partially hydrolyzed polyvinyl alcohol (degree of saponification 86.5 to 89.0 mol %)

Formulations in which the content of the partially hydrolyzed polyvinyl alcohol (degree of saponification 86.5 to 89.0 mol %) in the above standard O/W emulsion composition formulation was changed were made into O/W emulsion compositions by the same procedure as for the above standard formulation.

For the formulations in which the content of partially hydrolyzed polyvinyl alcohol was changed, it was ascertained whether it was possible to prepare a uniform formulation having a good feeling when used. Visual assessment was carried out by three people. The results are given in the table. Under the 'preparation' heading in the table, '○' denotes that it was possible to carry out uniform preparation, 'Δ' denotes that there was slight separation, and '×' denotes that it was not possible to carry out uniform preparation. Under the 'feeling when used' heading, '○' denotes that the formulation showed good spreading and had a good feeling when used, and 'Δ' denotes that there was a slightly sticky feel.

Under the '65°C/2W' heading, the properties after 2 weeks at 65°C were assessed. '○' denotes that there was no change from those immediately after being prepared.

**Table 5**

| | | | | | | | | | | |
|---|---|---|---|---|---|---|---|---|---|---|
| Content of polyvinyl alcohol % (degree of saponification 86.5 to 89.0 mol%) | 0.025 | 0.05 | 0.1 | 0.2 | 0.3 | 0.4 | 0.5 | 2.0 | 3.0 | 4.0 |
| Preparation | × | Δ | ○ | ○ | ○ | ○ | ○ | ○ | ○ | ○ |
| Feeling when used | - | - | ○ | ○ | ○ | ○ | ○ | ○ | ○ | Δ |
| 65°C/2W | - | - | ○ | ○ | ○ | ○ | ○ | ○ | ○ | - |

### Method for preparing lotion

0.5 g of carboxyvinyl polymer is dispersed in 25 g of purified water and heated to about 70°C. Furthermore, 1.0 g of partially hydrolyzed polyvinyl alcohol is dissolved in 25 g of purified water heated to about 70°C. The two aqueous solutions were combined, thus giving an aqueous phase. Separately, 13 g of medium chain fatty acid triglyceride and 10 g of 1,3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. The pH was adjusted to 5 to 7. A solution of magnesium chloride (appropriate amount) in the remaining purified water was added to the O/W emulsion composition, thus giving a lotion of the present invention.

Formulations in which the amount added of the partially hydrolyzed polyvinyl alcohol (degree of saponification 86.5 to 89.0 mol %) in the above standard lotion formulation was changed were made into lotions by the same procedure as for the above standard formulation.

For the formulations in which the amount of partially hydrolyzed polyvinyl alcohol added was changed, it was ascertained whether it was possible to prepare a uniform preparation having a good feeling when used. Visual assessment was carried out by three people. The results are given in the table. Under the 'properties' heading in the table, '○' denotes that a uniform formulation was obtained.

Under the '65°C/2W' heading, the properties after 2 weeks at 65°C were assessed.

**Table 6**

| | | | |
|---|---|---|---|
| Content of polyvinyl alcohol (%) (degree of saponification 86.5 to 89.0 mol%) | 0.1 | 0.2 | 3.0 |
| Viscosity (mPa·s) | 287 | 217 | 110 |
| pH | 5.26 | 5.06 | 4.82 |
| Properties | ○ | ○ | ○ |
| 65°C /2W | No change | No change | No change |

### (2) Test involving changing content of carboxyvinyl polymer

Formulations in which the content of carboxyvinyl polymer in the above standard O/W emulsion composition formulation was changed were made into O/W emulsion compositions by the same procedure.

For the formulations in which the content of carboxyvinyl polymer was changed, it was ascertained whether it was possible to prepare a uniform formulation having a good feeling when used. Visual assessment was carried out by three people. The results are given in the table. Under the 'properties' heading in the table, '○' denotes that a uniform preparation was obtained, 'Δ' denotes that there was slight separation, and '×' denotes that a uniform preparation could not be obtained. Under the 'feeling when used' heading, '○' denotes that the formulation spread well and had a good feeling when used, and 'Δ' denotes that there was a slightly sticky feel.

Under the '65°C/2W' heading, the properties after 2 weeks at 65°C were assessed. '○' denotes that it remained uniform from immediately after being prepared, and 'Δ' denotes that it became slightly nonuniform.

**Table 7**

| Type | Content % | 0.1 | 0.2 | 0.3 | 0.4 | 0.5 | 2.0 | 3.0 | 4.0 |
|---|---|---|---|---|---|---|---|---|---|
| Low viscosity (4000 to 10000 mPa·s (0.5%)) | Preparation | Δ | Δ | ○ | ○ | ○ | ○ | ○ | ○ |
| | Feeling when used | - | - | ○ | ○ | ○ | ○ | ○ | Δ |
| | 65°C/2W | - | - | ○ | ○ | ○ | ○ | Δ | Δ |
| | Content % | 0.05 | 0.1 | 0.15 | 0.2 | 0.3 | 1.0 | 2.0 | 3.0 |
| High viscosity (40000 to 60000 mPa·s (0.5%)) | Preparation | Δ | ○ | ○ | ○ | ○ | ○ | ○ | ○ |
| | Feeling when used | - | ○ | ○ | ○ | ○ | ○ | ○ | Δ |
| | 65°C/2W | - | ○ | ○ | ○ | ○ | ○ | ○ | Δ |

Formulations in which the content of carboxyvinyl polymer in the above standard lotion formulation was changed were made into lotions by the same procedure.

For the formulations in which the amount of carboxyvinyl polymer added was changed, it was ascertained whether it was possible to prepare a uniform formulation having a good feeling when used. Visual assessment was carried out by three people. The results are given in the table. Under the 'properties' heading in the table, '○' denotes that a uniform formulation was obtained.

Under the '65°C/2W' heading, the properties after 2 weeks at 65°C were assessed.

**Table 8**

| Content (%) | 0.1 | 3.0 |
|---|---|---|
| Type | High viscosity (40000 to 60000 mPa·s) | |
| Viscosity (mPa·s) | 80.8 | 229 |
| pH | 5.27 | 4.31 |
| Properties | ○ | ○ |
| 65°C/2W | No change | No change |

### (3) Test involving changing content of oil

Formulations in which the content of oil component in the above standard O/W emulsion composition formulation was changed were made into O/W emulsion compositions by the same procedure.

For the formulations in which the content of oil was changed, it was ascertained whether it was possible to prepare a uniform formulation having a good feeling when used. Visual assessment was carried out by three people. The results are given in the table. Under the 'preparation/feeling when used' headings in the table, '○' denotes that it was possible to carry out uniform preparation, and the preparation spread well and had a good feeling when used. 'Δ' denotes that there was slight separation and slightly sticky feel. '×' denotes that a uniform preparation could not be obtained.

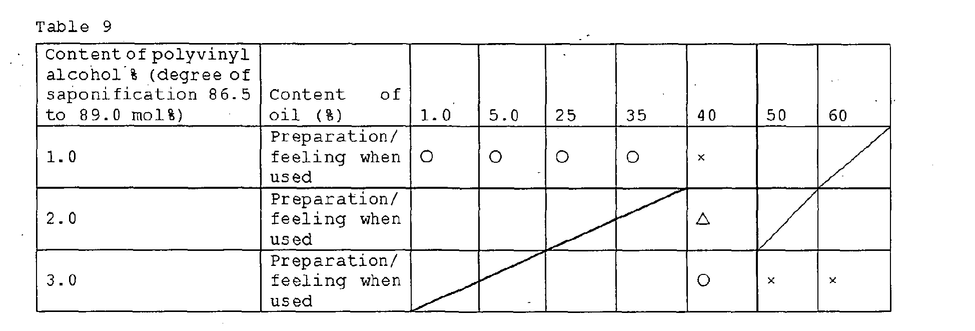

When the formulations that gave '○' under all headings were subjected to the 65°C/2W change over time test, none showed any change in properties.

Formulations in which the content of oil component in the above standard lotion formulation was changed were made into lotions by the same procedure.

For the formulations in which the content of oil was changed, it was ascertained whether it was possible to prepare a uniform preparation having a good feeling when used. Visual assessment was carried out by three people. The results are given in the table. Under the 'preparation/feeling when used' headings in the table, '○' denotes that it was possible to carry out uniform preparation.

**Table 10**

| | | |
|---|---|---|
| Content of oil (%) | 35 | 40 |
| Content of polyvinyl alcohol (%) (degree of saponification 86.5 to 89.0 mol%) | 1 | 3 |
| Viscosity (mPa·s) | 249 | 258 |
| pH | 4.85 | 4.70 |
| Properties | ○ | ○ |
| 65°C/2W (all headings) | No change | No change |

### Test Example 5 (Prototype Test 4: pH test)

In this test, O/W emulsion compositions in which the content of each component was changed were first prepared, and for these changed formulations, it was ascertained whether it was possible to prepare a uniform formulation having a good feeling when used. Following this, lotions in which the content of each component was changed were prepared, and it was ascertained whether it was possible to prepare a uniform formulation having a good feeling when used.

### Method for preparing O/W emulsion composition

0.5 g of carboxyvinyl polymer was dispersed in 25 g of purified water and heated to about 70°C, thus giving an aqueous solution. Furthermore, 1 g of partially hydrolyzed polyvinyl alcohol (degree of saponification 86.5 to 89.0 mol %) was dissolved in 25 g of purified water heated to about 70°C, thus giving an aqueous solution. The two aqueous solutions were combined, thus giving an aqueous phase. Separately, a mixture of 13 g of medium chain fatty acid triglyceride and 10 g of 1,3-butylene glycol was heated to about 70°C, and diisopropanolamine (appropriate amount) was dissolved therein, thus giving an oil phase. The oil phase was added to the aqueous phase, the remaining purified water was further added thereto, and this was stirred until uniform and subsequently cooled to room temperature while stirring, thus giving an O/W emulsion composition. This was used as a standard formulation.

Subsequently, the pH of the formulation was changed by changing the content of diisopropanolamine.

In these formulations, it was ascertained whether it was possible to prepare a uniform formulation having a good feeling when used. Visual assessment was carried out by three people. The results are given in the table. Under the 'preparation' heading in the table, '○' denotes that it was possible to carry out uniform preparation, and 'x' denotes that it was not possible to carry out uniform preparation.

Under the '65°C/2W' heading, the properties after 2 weeks at 65°C were assessed. '○' denotes that it remained uniform from immediately after being prepared.

**Table 11**

| | | | | | | | | | | | |
|---|---|---|---|---|---|---|---|---|---|---|---|
| pH | 3.1 | 3.5 | 4.0 | 5.3 | 5.8 | 6.2 | 6.9 | 7.7 | 8.9 | 9.3 | 9.7 |
| Preparation | × | ○ | ○ | ○ | ○ | ○ | ○ | ○ | ○ | ○ | ○ |
| 65°C/2W | - | ○ | ○ | ○ | ○ | ○ | ○ | ○ | ○ | ○ | ○ |

### Method for preparing lotion

0.5 g of carboxyvinyl polymer is dispersed in 25 g of purified water and heated to about 70°C. Furthermore, 1.0 g of polyvinyl alcohol is dissolved in 25 g of purified water heated to about 70°C. The two aqueous solutions were combined, thus giving an aqueous phase. Separately, 13 g of medium chain fatty acid triglyceride and 10 g of 1,3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. Subsequently the pH of the formulation was changed by changing the content of diisopropanolamine. A solution of magnesium chloride (appropriate amount) in the remaining purified water was added to the O/W emulsion composition, thus giving a lotion of the present invention.

For these formulations, it was ascertained whether it was possible to prepare a uniform formulation having a good feeling when used. Visual assessment was carried out by three people. The results are given in the table. Under the 'properties' heading in the table, '○' denotes that it was possible to carry out uniform preparation.

Under the '65°C/2W' heading, the properties after 2 weeks at 65°C were assessed.

**Table 12**

| | | |
|---|---|---|
| pH | 3.8 | 9.1 |
| Viscosity (mPa·s) | 79.4 | 261 |
| Properties | ○ | ○ |
| 65°C/2W (all headings) | No change | No change |

### Test Example 6 (Prototype Test 5: test involving changing content of liquid oil relative to total oil components)

In this test, O/W emulsion compositions in which the content of each component was changed were first prepared, and for these changed formulations, it was ascertained whether it was possible to prepare a uniform formulation having a good feeling when used. Subsequently, lotions in which the content of each component was changed were prepared, and it was ascertained whether it was possible to prepare a uniform formulation having a good feeling when used.

### Method for preparing O/W emulsion composition

0.5 g of carboxyvinyl polymer was dispersed in 2.5g of purified water and heated to about 70°C, thus giving an aqueous solution. Furthermore, 1 g of partially hydrolyzed polyvinyl alcohol (degree of saponification 86.5 to 89.0 mol %) was dissolved in 25 g of purified water heated to about 70°C, thus giving an aqueous solution. The two aqueous solutions were combined, thus giving an aqueous phase. Separately, a mixture of 13 g of medium chain fatty acid triglyceride and 10 g of 1,3-butylene glycol was heated to about 70°C, and diisopropanolamine (appropriate amount) was dissolved therein, thus giving an oil phase. The oil phase was added to the aqueous phase, the remaining purified water was further added, and this was stirred until uniform and subsequently cooled to room temperature while stirring, thus giving a cream. This was used as a standard formulation. The pH was adjusted to 5 to 7.

O/W emulsion compositions were prepared by the same procedure as for the above standard formulation except that, while keeping constant the total amount of oil components added in the standard O/W emulsion composition formulation, the medium chain fatty acid triglyceride was used as a liquid oil, the solid fat cetyl alcohol was used as the remaining oil component, and the content of liquid oil in the total oil components was changed.

For these changed formulations, it was ascertained wether it was possible to prepare a uniform formulation having a good feeling when used. Visual assessment was carried out by three people. The results are given in the table. Under the 'preparation' heading in the table, '○' denotes that it was possible to carry out uniform preparation. Under the 'properties' heading, '○' denotes that a uniform preparation was obtained, 'Δ' denotes that it was slightly nonuniform, and '×' denotes that it was nonuniform. Under the 'feeling when used' heading, '○' denotes that the formulation had a good feeling when used, 'Δ' denotes that there was slightly rough feel, and '×' denotes that there was a rough feel.

Under the '65°C/2W' heading, the properties after 2 weeks at 65°C were assessed. '○' denotes that it remained uniform from immediately after being prepared.

**Table 13**

| | | | | | | | |
|---|---|---|---|---|---|---|---|
| Content of liquid oil relative to total oils (%) | 10 | 30 | 40 | 50 | 60 | 70 | 90 |
| Preparation | ○ | ○ | ○ | ○ | ○ | ○ | ○ |
| Properties | × | × | Δ | Δ | ○ | ○ | ○ |
| Feeling when used | × | × | Δ | Δ | ○ | ○ | ○ |
| 65°C/2W | - | - | - | - | ○ | ○ | ○ |

### Method for preparing lotion

0.5 g of carboxyvinyl polymer was dispersed in 25 g of purified water and heated to about 70°C. Furthermore, 1.0 g of polyvinyl alcohol was dissolved in 25 g of purified water heated to about 70°C. The two aqueous solutions were combined, thus giving an aqueous phase. Separately, 13 g of medium chain fatty acid triglyceride and 10 g of 1,-3-butylene glycol were heated to about 70°C, and diisopropanolamine (appropriate amount) was dissolved therein, thus giving an oil phase. The oil phase was added to the aqueous phase, and this was stirred until uniform and subsequently cooled to room temperature while stirring, thus giving an O/W emulsion composition. The pH was adjusted to 5 to 7. A solution of magnesium chloride (appropriate amount) in the remaining purified water was added to the O/W emulsion composition, thus giving a lotion of the present invention.

Lotions were prepared by the same procedure except that, while keeping constant the total content of oil components in the above standard lotion formulation, the medium chain fatty acid triglyceride was used as a liquid oil, the solid fat cetyl alcohol was used as the remaining oil component, and the content of liquid oil in the total oil components was changed.

For these changed formulations, it was ascertained whether it was possible to prepare a uniform formulation having a good feeling when used. Visual assessment was carried out by three people. The results are given in the table. Under the 'preparation' heading in the table, 'o' denotes that it was possible to carry out uniform preparation. Under the 'properties' heading, 'o' denotes that a uniform preparation was obtained, 'Δ' denotes that it was slightly nonuniform, and '×' denotes that it was nonuniform. Under the 'feeling when used' heading, 'o' denotes that the formulation had a good feeling when used, 'Δ' denotes that there was slightly rough feel, and 'x' denotes that there was a rough feel.

Under the '65°C/2W' heading, the properties after 2 weeks at 65°C were assessed.

**Table 14**

| | | | | |
|---|---|---|---|---|
| Content of liquid oil relative to total oils (%) | 30 | 40 | 50 | 60 |
| Viscosity (mPa·s) | 280 | 234 | 309 | 233 |
| pH | 5.04 | 5.06 | 5.05 | 5.04 |
| Properties | × | Δ | Δ | ○ |
| Feeling when used | × | Δ | Δ | ○ |
| 65°C/2W | No change | No change | No change | No change |

### Test Example 7 Test for feeling when used Comparison of feeling when used between polyol being added and not being added

A comparison was made between Example 13, in which no polyol was added, and Example 14, in which polyol was added. A fixed amount of each emulsion lotion was applied to a forearm, and the feeling when used was evaluated using the evaluation criteria in the table below. The results are given in the table. The values in the table are the average values of three people subjected to the test.

### [Example 13]

| | |
|---|---|
| Medium chain fatty acid triglyceride | 13 mass % |
| Carboxyvinyl polymer | 0.5 mass % |
| Partially hydrolyzed polyvinyl alcohol | 1 mass % |
| Diisopropanolamine | appropriate amount |
| Magnesium chloride | 0.09 mass % |
| Purified water | total 100 mass % |
| pH 5.20 | |
| Viscosity 154 mPa·s | |

### [Example 14]

| | |
|---|---|
| Medium chain fatty acid triglyceride | 13 mass % |
| Carboxyvinyl polymer | 0.5 mass % |
| Partially hydrolyzed polyvinyl alcohol | 1 mass % |
| 1,3-Butylene glycol | 10 mass % |
| Diisopropanolamine | appropriate amount |
| Magnesium chloride | 0.09 mass % |
| Purified water | total 100 mass % |
| pH 5.10 | |
| Viscosity 169 mPa·s | |

**Table 15**

| Points | Feeling when used |
|---|---|
| 4 | Good |
| 3 | Slightly good |
| 2 | Fair |
| 1 | Slightly bad |
| 0 | Bad |

**Table 16**

| | Example 14 | Example 15 |
|---|---|---|
| Points | 2.3 Points | 3.7 Points |

The results of the test for feeling when used showed that adding a polyol reduced stickiness during application.

### INDUSTRIAL APPLICABILITY

In accordance with the present invention, an emulsion lotion composition that can be stored stably for a long period of time, causes little stimulation when used on the skin, and has high safety can be obtained. Furthermore, in accordance with the present invention, it is possible to easily obtain an emulsion lotion composition having an excellent feeling when used. This enables it to be used as an external preparation such as a pharmaceutical or a cosmetic.

## Claims

1. An emulsion lotion comprising:
a) a partially hydrolyzed polyvinyl alcohol having a degree of saponification of 70 to 96 mol %,
b) a carboxyvinyl polymer,
c) an oil component at not greater than 40 mass % relative to the total compositional amount, equal to or greater than 60 mass % of the contained oil component being liquid oil, and
d) an inorganic salt,
the pH being 3.5 to 8.5, and the viscosity at 25°C being 30 mPa·s to 310 mPa·s by oscillation viscometer.

2. The emulsion lotion according to Claim 1, wherein the inorganic salt is one type or two or more types selected from magnesium chloride, sodium chloride, ammonium chloride, potassium chloride, and calcium chloride.

3. The emulsion lotion according to Claim 1, containing substantially no surfactant.

4. The emulsion lotion according to Claim 1, wherein the liquid oil is polar liquid oil.

5. The emulsion lotion according to Claim 4, wherein the polar liquid oil is synthetic oil having an IOB value of equal to or greater than 0.1.

6. The emulsion lotion according to Claim 4, wherein the polar liquid oil is vegetable oil having a fatty acid triglyceride as a main component.

7. The emulsion lotion according to any one of Claims 1 to 6, which is a composition for external application.

8. The emulsion lotion according to any one of Claims 1 to 6, further comprising a polyol.
